# EUROPÄISCHE PATENTANMELDUNG

(11) **EP 2 700 940 A1**
(43) Veröffentlichungstag der Anmeldung: **26.02.2014**
(21) Anmeldenummer: 12181119.4
(22) Anmeldetag: 21.08.2012
(51) Int. Cl.: G01N 33/18

(54) **Verfahren und Vorrichtung zur Messung des Gasgehalts in einer Flüssigkeit sowie Verwendung einer solchen Vorrichtung**

(71) Anmelder: Siemens Aktiengesellschaft, 80333 München (DE)
(72) Erfinder: Mattejat, Arno, 91056 Erlangen (DE)

(57) **Zusammenfassung**

Eine schnelle und zuverlässige Kontrolle des Gasgehalts einer Flüssigkeit (K) wird durchgeführt, indem
- zumindest eine Teilmenge (M) der Flüssigkeit (K) in eine Messzelle (12) gefördert wird,
- in der Messzelle (12) ein Unterdruck eingestellt wird,
- die Teilmenge (M) mit einem wellenförmigen Messsignal bestrahlt wird,
- das wellenförmige Messsignal nach dem Kontakt mit der Teilmenge (M) der Flüssigkeit (K) von mindestens einem Detektor (8, 10) gemessen wird,
- ein Trübungswert (TW) der Flüssigkeit (K) gemessen und mit einem Referenzwert (R) verglichen wird, und
- wenn durch den Vergleich mit dem Referenzwert (R) der Eintritt einer Trübung der Flüssigkeit (K) detektiert wird, ein Druck (p) und eine Temperatur (T) in der Messzelle gemessen werden und daraus der Gasgehalt in der Flüssigkeit (K) bestimmt wird.

## Beschreibung

Die Erfindung betrifft ein Verfahren zur Messung des Gasgehalts in einer Flüssigkeit. Die Erfindung betrifft weiterhin eine Vorrichtung zur Messung des Gasgehalts in einer Flüssigkeit sowie eine Verwendung einer solchen Vorrichtung.

In vielen technischen Bereichen ist eine Messung des Gasgehalts in Betriebsflüssigkeiten erforderlich, da das Vorliegen von Gas die Eigenschaften dieser Flüssigkeiten beeinflusst. Eine Messung des Gasgehalts ist daher z.B. für die Dimensionierung und Konstruktion von Maschinenteilen und zur Entwicklung von Flüssigkeiten mit günstigem Gasabscheideverhalten relevant. Insbesondere ist es wichtig, den Gasgehalt einer Schmierstoffflüssigkeit oder einer Heiz- bzw. Kühlflüssigkeit zu kennen.

Die Messung von Gas in Flüssigkeiten ist beispielsweise von Bedeutung für den störungsfreien Betrieb einer elektrochemischen Zelle, wie z.B. einer Brennstoffzelle. Im Betrieb einer Brennstoffzelle muss entstehende Verlustwärme aus dem aktiven Bereich der Brennstoffzelle weitgehend entfernt werden, um lokale Überhitzungen (sogenannte "hot spots") zu vermeiden. Dies erfolgt am effektivsten durch ein flüssiges Kühlmittel, das die Brennstoffzelle durchströmt.

In einem Brennstoffzellenstapel mit Brennstoffzellen basierend auf der Polymer-Elektrolyt-Membran (PEM)-Technologie befinden sich die Elektroden an der einer Elektrolyt-Membran bzw. Katalysatorschicht abgewanden Seite im Kontakt mit jeweils einer so genannten Bipolarplatte oder Kühleinheit. Die Bipolarplatte hat die Aufgabe, die einzelnen Brennstoffzellen (medienseitig) zu trennen, für Stromfluss im Zellenstapel zu sorgen und die Reaktionswärme zu entfernen.

Neben der Stromleitung und der Führung der Reaktanten Wasserstoff und Sauerstoff, erfüllen die Bipolarplatten ihre Kühlfunktion, indem ein Kühlmittel, insbesondere Wasser, durch diese geleitet wird. Wegen seiner hohen spezifischen Wärmekapazität, der geringen elektrischen Leitfähigkeit, der guten Medienverträglichkeit und der niedrigen Betriebskosten kommt bei PEM-Brennstoffzellen hauptsächlich deionisiertes Wasser (Deionat) als Kühlmittel zum Einsatz. Das Kühlmittel darf nur eine sehr geringe Leitfähigkeit haben und sollte möglichst gasfrei sein, damit sich keine Gasblasen an der Oberfläche der Bipolarplatten festsetzen, da Bereiche mit Gasblasen schlechter gekühlt werden können und daher lokal überhitzt werden. Aufgrund physikalischer und konstruktiver Vorgaben ist jedoch nicht auszuschließen, dass auch ursprünglich gasfreies Kühlwasser bei seinem Einsatz Gas aufnimmt und mit Blasen versehen ist.

In großen Heizungsanlagen werden sog. Vakuum-Entgaser eingesetzt, um gelöste Luft aus dem Heizungswasser zu entfernen. Ähnliche Einrichtungen können auch in Brennstoffzellenanlagen benutzt werden, um Gas aus dem Kühlwasser zu entfernen. Ziel dieser Methoden ist, kein Zwei-Phasen-Gemisch (Gas/Wasser) im Kühlwasser zuzulassen, so dass eine Gasansammlung und damit eine lokale Überhitzung an der Bipolarplatte nicht auftreten können.

Der Erfindung liegt die Aufgabe zugrunde, eine schnelle und zuverlässige Kontrolle des Gasgehalts einer Flüssigkeit, z.B. eines Kühlmittels, zu ermöglichen.

Die Aufgabe wird erfindungsgemäß gelöst durch ein Verfahren zur Messung eines Gasgehalts in einer Flüssigkeit, wobei
- zumindest eine Teilmenge der Flüssigkeit in eine Messzelle gefördert wird,
- in der Messzelle ein Unterdruck eingestellt wird,
- die Teilmenge mit einem wellenförmigen Messsignal bestrahlt wird,
- das Messsignal nach dem Kontakt mit der Teilmenge gemessen wird,
- ein Trübungswert der Flüssigkeit bestimmt wird und mit einem Schwellwert verglichen wird, und
- wenn der Trübungswert größer oder gleich dem Schwellwert ist, ein Druck und eine Temperatur in der Messzelle gemessen werden und mit Hilfe der Druck- und Temperaturmessung der Gasgehalt in der Flüssigkeit ermittelt wird.

Die Aufgabe wird weiterhin erfindungsgemäß gelöst durch eine Vorrichtung zur Messung eines Gasgehalts in einer Flüssigkeit, umfassend:
- eine Messzelle zur Aufnahme zumindest einer Teilmenge der Flüssigkeit,
- Mittel zum Erzeugen von Unterdruck in der Messzelle,
- ein Messsignalsender zum Bestrahlen der Teilmenge mit einem wellenförmigen Messsignal,
- mindestens einen Detektor zum Messen des Messsignals nach dem Kontakt mit der Teilmenge in der Messzelle,
- Sensoren zum Bestimmen eines Druck und einer Temperatur in der Messzelle,
- sowie eine Analyse- und Steuereinheit, die dafür eingerichtet ist einen Trübungswert der Flüssigkeit zu bestimmen und diesen mit einem Schwellwert zu vergleichen und wenn der Trübungswert größer oder gleich dem Schwellwert ist, mit Hilfe der Druck- und Temperaturmessung in der Messzelle den Gasgehalt in der Flüssigkeit zu ermitteln.

Die Aufgabe wird schließlich erfindungsgemäß gelöst durch eine Verwendung einer derartigen Vorrichtung zur Messung des Gasgehaltes in einem Kühlmittel einer elektrochemischen Zelle, insbesondere einer Brennstoffzelle.

Die in Bezug auf das Verfahren nachstehend aufgeführten Vorteile und bevorzugten Ausführungen lassen sich sinngemäß auf die Vorrichtung und ihre Verwendung übertragen.

Die Erfindung beruht auf der Erkenntnis, dass bei der Entspannung einer Flüssigkeit mit gelöstem Gas, das Gas in Form von kleinen Blasen ausfällt, die zu einer Trübung der Flüssigkeit führen. Unter Trübung wird hierbei eine Veränderung der Transmission und der Reflektion der Teilmenge für Wellen verstanden, die durch eine Veränderung der Zusammensetzung Flüssigkeit/Gas der Teilmenge hervorgerufen wird. Quantitativ wird diese Veränderung durch den Trübungswert ausgedruckt. Um bestimmen zu können, ob Gas enthalten ist, ist es somit lediglich erforderlich, die Trübung der Flüssigkeit bei der Entspannung der Flüssigkeit in der Messzelle zu detektieren. Zudem sind Sensoren erforderlich, mit deren Hilfe der Druck und die Temperatur in der Messzelle bestimmt werden.

Vorzugsweise wird die Teilmenge mit einem elektromagnetischen Messsignal bestrahlt, insbesondere mit Licht. Hierfür sind eine Lichtquelle als Messsignalsender und mindestens einer Lichtdetektor vorgesehen. Die Messezelle ist dabei durchlässig für das Licht der Lichtquelle.

Alternativ wird die Teilmenge bevorzugt mit einem akustischen Messsignal, insbesondre mit Ultraschall bestrahlt. In diesem Fall sind ein Ultraschallsender und ein Ultraschalldetektor erforderlich.

Der ermittelte Trübungswert stellt ein Maß für die Veränderung der Zusammensetzung der Flüssigkeit mit sinkendem Druck dar. Der Trübungswert wird insbesondere durchgehend oder in Zeitabständen von wenigen Sekunden bis zu vielen Stunden bestimmt und mit einem Schwellwert verglichen. Der Schwellwert definiert dabei das Eintreten der Trübung in der Flüssigkeit. Wenn bei der Messung somit der Trübungswert den Schwellwert erreicht hat oder diesen überschreitet, ist eine Trübung der Flüssigkeit detektiert, die qualitativ auf das Vorliegen von Gas in der Flüssigkeit deutet. Quantitativ wird der Gasgehalt durch die Druck- und die Temperaturmessung in der Messzelle beim Eintreten der Trübung bestimmt. Aufgrund von vorhandenen Kenndaten kann aus der Temperatur und dem Druck, bei denen die Trübung einsetzt, auf die Menge des gelösten Gases geschlossen werden. Beispielsweise deutet ein frühes Eintreten der Trübung bei einem Druck, der knapp unter dem Umgebungsdruck ist, auf eine große Menge an Gas.

Wenn bei der Messung Gas festgestellt wird, wird insbesondere ein entsprechendes Signal ausgegeben, das an eine Steuereinheit oder an eine optische Anzeige weitergeleitet wird. Mit diesem Signal wird insbesondere ein Vakuum-Entgaser eingeschaltet und solange betrieben, bis die Flüssigkeit wieder gasfrei ist.

Beim Einsatz der Vorrichtung zur Messung eines Gasgehalts in einer elektrochemischen Zelle wie z.B. einer Brennstoffzelle kann die Messzelle dabei an einer Neben- oder Bypassleitung parallel zu einer Hauptleitung für den Kühlmittelkreislauf angeordnet sein, so dass lediglich ein kleiner Teil des Kühlmittels durch die Messzelle durchgeleitet wird. Alternativ kann die Messzelle in der Hauptleitung integriert sein, so dass ein Umleiten des Kühlmittels zu Messzwecken nicht erforderlich ist.

Die Überwachung des Gasgehalts in der Flüssigkeit kann diskontinuierlich erfolgen, indem aus dem Hauptstrom in der Hauptleitung eine kleine Teilmenge durch eine Pumpe in die Messzelle gefördert wird und nach dem Schließen eines Einlassventils die Pumpe so gesteuert wird, dass der Druck in der Messzelle langsam abfällt und die optische Messung durchgeführt wird.

Alternativ wird die Flüssigkeit jedoch kontinuierlich in die Messzelle zur Messung des Trübungswerts eingeleitet. Hierfür ist bevorzugt ein Regelventil zum kontinuierlichen Zufluss von Kühlmittel in die Messzelle vorgesehen. Zur Druckabsenkung in der Messzelle wird das Regelventil derart angesteuert, dass die gewünschten, temperaturabhängigen Druckwerte, die für die Messung benötigt werden, bei konstanter Pumpendrehzahl eingestellt werden. Der Druck in der Messzelle sinkt langsam, bis sich eine Trübung einstellt. Aus der zu diesem Zeitpunkt gemessenen Druck und Temperatur lässt sich die Gasbeladung ermitteln. Grenze zur Durchführung des Verfahrens ist ein Druck kurz oberhalb des Siededrucks (ca. 50 mbar oberhalb des Siededrucks). Oberhalb dieser Druckgrenze kann durch das Auftreten der Trübung festgestellt werden, ob in der Flüssigkeit Gas enthalten ist.

Eine weitere Alternative stellen ein festes Drosselventil und ein Absenken des Drucks durch die Pumpe dar. Die Auswertung erfolgt wie im obigen Absatz beschrieben.

Ein Vorteil der oben beschriebenen Messung ist, dass mit kleinen Volumina bzw. Teilmengen an Flüssigkeit und minimalen Mengen an ausfallendem Gas gearbeitet wird, so dass die Messung durch eine hohe Empfindlichkeit gekennzeichnet ist. Ein weiterer Vorteil ist, dass sie voll automatisch und ohne den Eingriff eines Bedieners ablaufen kann. Wenn der Gasgehalt eines Kühlmittels für eine elektrochemische Zelle gemessen wird, wird zudem die geprüfte Teilmenge nicht entsorgt, sondern sie wird nach der Messung der Trübung insbesondere in den Kühlmittelkreislauf der elektrochemischen Zelle zurück gepumpt.

Gemäß einer bevorzugten Ausführung werden der Druck und/oder die Temperatur in der Messzelle derart angesteuert, dass sie oberhalb des Siedepunkts bleiben. Der Siedepunkt ist hierbei als ein Punkt an der Phasengrenze flüssig/gasförmig im Phasendiagramm (Dampfdruckkurve) der Flüssigkeit definiert, in dem die Sättigungstemperatur bzw. Siedetemperatur und der Sättigungsdampfdruck bzw. Siededruck erreicht ist. Insbesondere wird der Druck in der Messzelle derart angesteuert, dass er während der Messung bei der entsprechenden Temperatur etwa 50 mbar oberhalb des Siededrucks bleibt. Beim Herabsetzen des Drucks in der Messzelle verdampft die Flüssigkeit in Form kleiner Blasen, wenn bei der entsprechenden Temperatur der Siededruck erreicht ist. Derartige Dampfblasen haben den gleichen Einfluss auf die Trübung wie ein in der Flüssigkeit gelöstes Gas. Um eine Verfälschung der Messergebnisse für den Trübungswert aufgrund von Siedeblasen zu vermeiden, werden die Temperatur- und Drucksensoren benötigt, durch deren Signale unterschieden werden kann, ob es sich bei der Trübung um einen Effekt aus der Ausgasung oder aus der Verdampfung der Flüssigkeit handelt. Daher ist es vorgesehen, dass während der Durchführung des Verfahrens der Druck und die Temperatur insbesondere kontinuierlich oder in kurzen Zeitabständen gemessen werden, um rechtzeitig auf eine Annäherung an den Siedepunkt reagieren zu können.

Gemäß einer bevorzugten Variante wird der Schwellwert als Zahlenwert vorgegeben. Der Schwellwert kann dabei ein in der Analyseeinheit hinterlegter Wert sein, kann jedoch auch vor jeder Messung neu eingegeben werden.

Um Veränderungen der Flüssigkeit zu erkennen, welche nicht durch den Gasgehalt in der Teilmenge hervorgerufen sind, welche jedoch die Transmission- und Reflektionseigenschaften der Flüssigkeit beeinflussen, z.B. Verfärbungen oder Verschmutzungen oder Veränderungen an Messkomponenten (beispielsweise durch eine Schwächung der Lichtquelle), wird zweckdienlicherweise eine Lichtmessung zur Festlegung eines Referenzwertes durchgeführt bevor der Unterdruck eingestellt wird (d.h. noch bei Systemdruck). Der Referenzwert wird zum Beginn, bei noch nicht abgesenktem Druck in der Messzelle insbesondere über eine Licht- oder Ultraschallmessung gewonnen und definiert einen Anfangszustand der Flüssigkeit im Hinblick auf ihre Trübung. Eine Veränderung des Trübungswerts gegenüber dem Referenzwert würde somit auf das Vorliegen von Gas in der Flüssigkeit hindeuten.

Vorzugsweise wird der Referenzwert, sobald er ermittelt ist, mit einem Grenzwert verglichen. Aus diesem Vergleich wird eine Selbstüberwachung der Vorrichtung abgeleitet, mit deren Hilfe sich Verschmutzungen oder Geräteausfälle erkennen lassen. Wenn bei Umgebungsdruck eine Trübung der Flüssigkeit durch den Vergleich des Referenzwerts mit dem Grenzwert festgestellt wird, deutet dies insbesondere auf eine deutliche Verschmutzung der Messvorrichtung. Bei einem vordefinierten Schwellwert kann der Grenzwert durch den Schwellwert gebildet sein oder eine Funktion des Schwellenwerts sein (z.B. der Grenzwert entspricht 80 % des Schwellwerts). Im Normalfall liegt der Referenzwert unterhalb des Grenzwerts, d.h. die Flüssigkeit ist bei Umgebungs- bzw. Systemdruck "klar".

Alternativ zum vorgegebenen Zahlenwert für den Schwellwert wird der Schwellwert gemäß einer weiteren bevorzugten Variante auf Grundlage des gemessenen Referenzwertes berechnet. Um den Schwellwert zu bilden, wird hierbei insbesondere zum Referenzwert ein Toleranzbereich Δ addiert, durch welches eine Erhöhung der Trübung quantitativ ausgedrückt ist. Wenn der Trübungswert den Referenzwert um den Toleranzbereich Δ übersteigt (d.h. wenn der Trübungswert den Schwellwert erreicht und größer als der Schwellwert ist), liegt eine Trübung der Flüssigkeit vor.

Zur Bestimmung des Trübungswerts wird bevorzugt eine Intensität des Messsignals nach dem Kontakt mit der Flüssigkeit in der Messzelle herangezogen. Je intensiver die Trübung, desto größer ist die Gasmenge in der Flüssigkeit.

Nach einer bevorzugten Variante wird ein durch die Flüssigkeit transmittierter Anteil des wellenförmigen Messsignals gemessen, indem der Detektor derart angeordnet ist, dass die Messzelle sich zwischen dem Messsignalsender und dem Detektor befindet. Nach einer alternativen Variante wird ein von der Flüssigkeit reflektierter Anteil des Messsignals gemessen, indem der Detektor an der Seite der Messzelle angeordnet ist, an der sich auch der Messsignalsender befindet. Möglich sind auch Messsysteme, bei denen sowohl das transmittierte als auch das reflektierte Messsignal gemessen werden. Dabei wird berücksichtigt, dass Gasblasen innerhalb der Flüssigkeit die Intensität des durchgehenden Lichtes abschwächen und somit den Anteil des gestreuten Messsignals erhöhen, so dass der Trübungswert steigt. Eine Alterung des Messsignalsenders sowie eine Verschmutzung der lichtdurchlässigen Messzelle schwächt die Intensität der durchgehenden Wellen des Messsignals und des gestreuten Messsignals, d.h. der Trübungswert bleibt konstant.

Im Hinblick auf eine störungsfreie Messung wird eine Geschwindigkeit der Flüssigkeit derart geregelt, dass Gasblasen von einer Wand der Messzelle ausgeschwemmt werden. Dadurch wird eine ungünstige Beeinflussung der Messergebnisse, insbesondere bei einem kontinuierlichen Durchlauf von Teilmengen der Flüssigkeit durch die Messzelle, vermieden.

Ein Ausführungsbeispiel der Erfindung wird anhand einer Zeichnung näher erläutert. Hierin zeigen:
- FIG 1: eine Vorrichtung zur diskontinuierlichen Überwachung des Gasgehalts eines Kühlmittels einer Brennstoffzelle,
- FIG 2: eine Vorrichtung zur kontinuierlichen Überwachung des Gasgehalts eines Kühlmittels einer Brennstoffzelle, und
- FIG 3: eine dritte Ausführungsvariante einer Vorrichtung zum Messen des Gasanteils in einer Flüssigkeit, und
- FIG 4: eine Anordnung einer Messzelle.

Gleiche Bezugszeichen haben in den verschiedenen Figuren die gleiche Bedeutung.

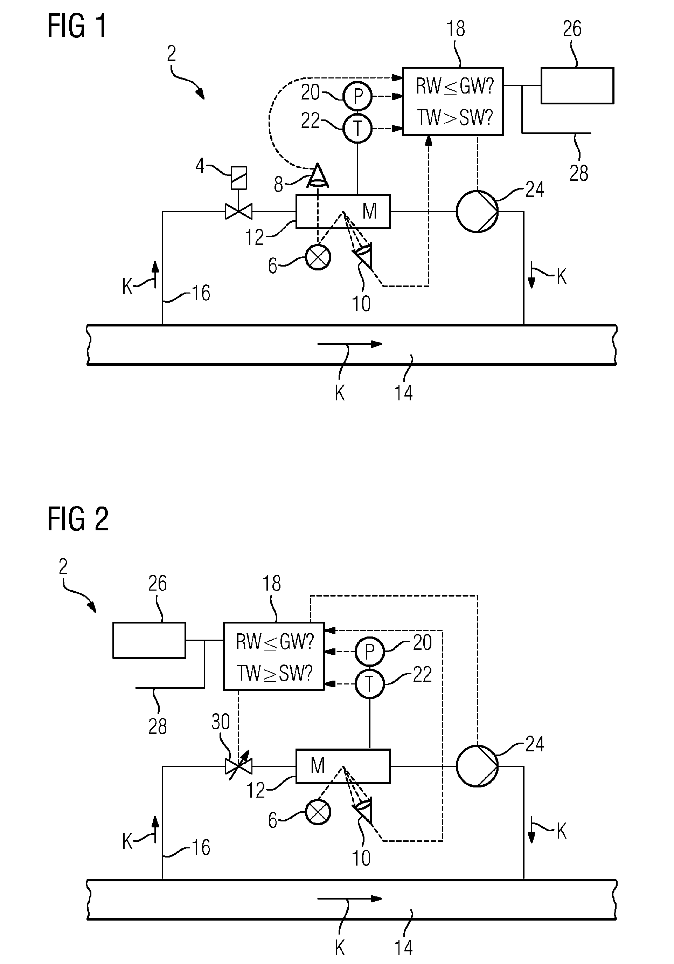

In FIG 1 ist eine erste Vorrichtung 2 zur Messung des Gasgehalts eines flüssigen Kühlmittels K, hier ein Kühlwasser für eine nicht näher gezeigte Brennstoffzelle, gezeigt. Die Vorrichtung 2 umfasst ein Einlassventil 4, ein Messsignalsender 6 für ein wellenförmiges Messsignal, der im gezeigten Ausführungsbeispiel eine Lichtquelle ist, zwei Detektoren 8, 10, die symbolisch als offene Augen dargestellt sind, sowie eine für das Licht der Lichtquelle 6 durchlässige Messzelle 12. Als Lichtquelle 6 eigenen sich LED, Glühlampen, Gasentladungslampen, etc.. Jeder der Lichtdetektoren kann z.B. Fototransistoren, Fotodioden oder Fotowiderstände umfassen.

Alternativ zur Lichtmessung kann der Messsignalsender 6 ein hier nicht näher gezeigter Ultraschallsender sein, entsprechend ist dann der Detektor 8, 10 ein Ultraschalldetektor.

Im gezeigten Ausführungsbeispiel wird mit Hilfe der Lichtdetektoren 8, 10 ein Licht aus der Lichtquelle 6 nach dem Kontakt mit dem Kühlwasser K in der Messzelle 12 gemessen. Ein erster Lichtdetektor 8 ist dabei für eine Messung des durch das Kühlwasser K in der Messzelle 12 transmittierten Lichts vorgesehen und ist dabei derart angeordnet, dass die Messzelle 12 sich zwischen ihm und der Lichtquelle 6 befindet. Ein zweiter Lichtdetektor 10 ist an der gleichen Seite der Messzelle 12 wie die Lichtquelle 6 angeordnet und misst dabei das von der Teilmenge M am Kühlwasser K reflektierte Licht.

Die Vorrichtung 2 ist parallel zu einer Hauptleitung 14 für das Kühlwasser K angeordnet. Über eine Nebenleitung 16 wird im offenen Zustand des Einlassventils 4 eine Teilmenge M an Kühlwasser K aus der Hauptleitung 14 in die Messzelle 12 gefördert. Wenn die Messzelle 12 insbesondere vollständig mit Kühlwasser K aufgefüllt ist, wird das Einlassventil 4 geschlossen. Anschließend wird eine Referenzmessung durchgeführt, bei der ein Referenzwert RW für die Trübung des Kühlwassers K gewonnen wird. Mit Trübung wird hierbei eine Veränderung der Transmissions- bzw. Reflektionsverhalten der Flüssigkeit aufgrund einer Bildung von Gasblasen in der Messzelle bezeichnet. Der Referenzwert RW wird in einer Analyse- und Steuereinheit 18, die ebenfalls Teil der Vorrichtung 2 ist, hinterlegt. Im gezeigten Ausführungsbeispiel ist lediglich eine Einheit zur Analyse der Messergebnisse und zur Steuerung der Komponenten der Vorrichtung 2 vorgesehen, alternativ können auch zwei separate Einheiten diese zwei Funktionen übernehmen.

Um den Einfluss von Verfärbungen oder Verschmutzungen im Kühlwasser K oder den Einfluss einer Alterung bzw. Schwächung der Lichtquelle 6 auf die Messung zu minimieren oder gar zu unterbinden, wird der Referenzwert RW in der Analyse- und Steuereinheit 18 mit einem vorgegebenen, fixen Grenzwert GW verglichen. Wenn der Referenzwerts RW dem Grenzwert GW entspricht oder diesen überschreitet, wird die Funktionalität der Vorrichtung 2 überprüft. Auf diese Weise erfolgt eine Selbstüberwachung der Vorrichtung 2, bei der Verschmutzungen oder Geräteausfälle frühzeitig erkannt werden. Im Normalfall ist der Referenzwert RW kleiner als der Grenzwert GW, dann ist das Kühlwasser K klar genug, um die Messung durchzuführen.

Nach der Referenzmessung wird ein Druck p in der Messzelle 12 herabgesetzt. Der Druck p sowie die Temperatur T in der Messzelle 12 werden dabei mit Hilfe eines Drucksensors 20 und eines Temperatursensors 22 überwacht. Wenn das Kühlwasser K gelöstes Gas enthält, fällt dieses Gas bei der Entspannung des Kühlwassers K in der Messzelle 12 in Form von kleinen Blasen aus, die zu einer Trübung des Kühlwassers K führen. Da allerdings beim Erreichen des Siedepunkts (Siededruck und Siedetemperatur) das Kühlwasser K selbst in Form kleiner Blasen verdampft, die das Messergebnis für die Trübung beeinflussen, werden der Druck p und gegebenenfalls die Temperatur T in der Messzelle 12 derart geregelt, dass sie stets oberhalb des Siedepunkts bleiben, insbesondere mindestes 50 mbar oberhalb des Siedepunkts bleiben.

Auf Grundlage der Lichtmessung bei Unterdruck wird ein Trübungswert TW für das Kühlwasser K in der Messzelle 12 ermittelt. Für die Ermittlung des Trübungswerts TW reicht es aus, wenn nur ein Lichtdetektor 8, 10 vorgesehen ist, der entweder das durchgelassene oder das reflektierte Licht detektiert. Für die Bestimmung des Trübungswerts TW wird insbesondere die Intensität des Lichts nach dem Kontakt mit dem Kühlwasser K in der Messzelle 12 herangezogen.

Der ermittelte Trübungswert TW wird der Analyse- und Steuereinheit 18 zugeführt und mit einem Schwellwert SW verglichen. Der Schwellwert SW kann als Zahlwert vorgegeben sein, der Schwellwert SW kann jedoch alternativ Messung spezifisch definiert sein, indem er vom Referenzwert RW abhängig ist. Wenn der Trübungswert TW innerhalb eines vorgegebenen Toleranzbereichs bleibt und den Schwellwert SW nicht erreicht, lässt sich daraus schließen, dass im Kühlwasser K keine Gasblasen enthalten sind. In diesem Fall wird die Teilmenge M an Kühlwasser K, welche in der Messzelle 12 gemessen wurde, über eine Pumpe 24 zurück in die Hauptstromleitung 14 geführt. Die Pumpe 24 wird insbesondere von der Analyse- und Steuereinheit 18 angesteuert.

Wenn jedoch der Trübungswert TW den Schwellwert SW erreicht hat oder diesen überschreitet, ist dies ein Zeichen dafür, dass im Kühlwasser K Gas enthalten ist. Um Informationen über die Menge an Gas im Kühlwasser K zu erhalten, wird dabei berücksichtigt, bei welchem Druck p und welcher Temperatur T die Trübung des Kühlwassers K in der Messzelle 12 auftritt. Die Anzeige 26 kann dabei dazu verwendet werden, den Gasanteil im Kühlwasser K anzuzeigen. Ergänzend dazu kann über die Anzeige 26 z.B. auch das Ergebnis des Vergleichs des Trübungswerts TW mit dem Schwellenwert SW dargestellt werden. Bei detektiertem Gas im Kühlwasser K, d.h. beim Eintreten einer Trübung in der Messzelle 12, wird außerdem ein Steuersignal 28 ausgegeben, welches veranlasst, dass eine Entgasungsvorrichtung, insbesondere ein hier nicht näher gezeigter, der Vorrichtung 2 nachgeschalteter Vakuum-Entgaser, aktiviert wird. Nach der Messung in der Messzelle 12 wird dabei auch die Teilmenge M an Kühlwasser K anschließend im Vakuum-Entgaser aufbereitet.

Die Vorrichtung 2 zur Überwachung des Gasgehalts im Kühlwasser K gemäß FIG 2 unterscheidet sich von der Vorrichtung 2 gemäß FIG 1 im Wesentlichen dadurch, dass die Anordnung im zweiten Ausführungsbeispiel für eine kontinuierliche Messung geeignet ist. Hierfür ist das Einlassventil 4 durch ein Regelventil 30 ersetzt. Das Regelventil 30 wird von der Analyse- und Steuereinheit 18 angesteuert, um den Zufluss von Kühlwasser K in die Messzelle 12 zu regulieren. Bei konstanter Drehzahl der Pumpe 24 wird das Regelventil 30 langsam geschlossen. Dabei sinkt der Druck in der Messzelle 12, bis sich Gasblasen bilden. Die Grenze für die Messung des Gasgehalts in der Teilmenge M liegt dabei bei ca. 50 mbar oberhalb des Siededrucks. Durch das Eintreten einer Trübung in der Messzelle 12 im Messbereich kann somit festgestellt werden, ob im Kühlwasser K Gas enthalten ist. Durch die weitere Auswertung des Drucks p und der Temperatur T beim Eintreten der Trübung kann zudem der Gasgehalt gemessen werden.

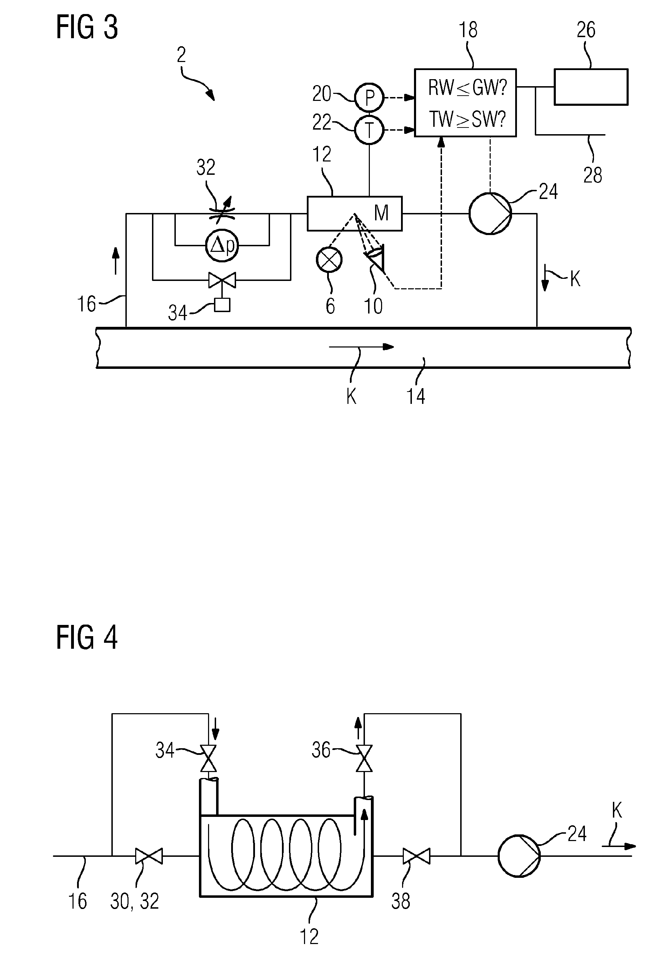

Gemäß FIG 3 strömt das Kühlwasser K durch ein fixes Drosselventil 32 in die Messzelle 12. Die Pumpendrehzahl wird so geändert, dass sich in der Messzelle 12 ein immer niedrigerer Druck einstellt, bis Trübung eintritt. Die Messung beginnt, wenn über dem Drosselventil 32 insbesondere ein Druckunterschied von mindestens 100 mbar entsteht.

In allen gezeigten Figuren erfolgt die Messung des Trübungswerts TW mit Hilfe einer Teilmenge M an Kühlwasser K, welche durch die Nebenleitung 16 umgeleitet wird. Alternativ wird die Vorrichtung 2 zur Überwachung des Gasgehalts im Kühlwasser K direkt in der Hauptleitung 14 integriert, so dass der gesamte Kühlmittelstrom durch die Vorrichtung 2 zu Messzwecken geleitet wird.

Die Messung wird in der Regel bei einem Differenzdruck von ca. 100 bar unter dem Anfangsdruck gestartet.

Darüber hinaus ist in allen gezeigten Ausführungen vor der Messzelle 12 ein Spülventil 34 mit großem Durchmesser vorgesehen, welches nur in FIG 3 gezeigt ist. Wenn sich Gasblasen an der Wand der Messzelle 12 festgesetzt haben, werden diese mit Hilfe des Spülventils 34, das einen großen Durchfluss zulässt, weggeschwemmt. Eine Verfälschung der Messergebnisse wird somit vermieden.

Die Spülung der Messzelle 12 wird anhand FIG 4 im Detail erläutert. Die Messzelle 12 wird ergänzt durch zwei Anschlüsse, die eine Strömung innerhalb der Messzelle 12 erzeugen, die sich schraubenförmig um die Mittelachse windet. Dies kann erreicht werden, wenn die Anschlüsse für den Spülzyklus radial angeordnet werden wie in FIG 4 gezeigt. Dazu sind außer dem bereits berücksichtigten Spülventil 34 zwei weitere Ventile 36, 38 erforderlich, die zwischen Spülung und Messung umschalten.

Für die Messung wird das Spülventil 34 bzw. 36 geschlossen und das Ventil 38 geöffnet. Dabei entsteht eine axiale Strömung durch die Messzelle 12.

Zur Reinigung der Wände von anhaftenden Blasen werden die Spülventile 34 und 36 geöffnet, Ventil 38 geschlossen und es entsteht eine rotierende Strömung. Durch die Rotation des Messzelleninhalts erreicht man an der Wand eine höhere Strömungsgeschwindigkeit, als wenn die Messzelle 12 bei gleichen Ventildurchmessern axial durchströmt würde. Blasen werden dadurch besser fortgeschwemmt. Für Spülventile 34, 36 können bei radialer Durchströmung dann kleinere Durchmesser verwendet werden, als bei rein axialer Durchströmung der Messzelle 12. Alternativ könnte das Spülventil 34 durch ein Drosselventil oder ein Regelventil ersetzt werden.

## Patentansprüche

1. Verfahren zur Messung eines Gasgehalts in einer Flüssigkeit (K), wobei
- zumindest eine Teilmenge (M) der Flüssigkeit (K) in eine Messzelle (12) gefördert wird,
- in der Messzelle (12) ein Unterdruck eingestellt wird,
- die Teilmenge (M) mit einem wellenförmigen Messsignal bestrahlt wird,
- das Messsignal nach dem Kontakt mit der Teilmenge (M) gemessen wird,
- ein Trübungswert (TW) der Flüssigkeit (K) bestimmt wird und mit einem Schwellwert (SW) verglichen wird, und
- wenn der Trübungswert (TW) größer oder gleich dem Schwellwert (SW) ist, ein Druck (p) und eine Temperatur (T) in der Messzelle (12) gemessen werden und mit Hilfe der Druck- und Temperaturmessung der Gasgehalt in der Flüssigkeit (K) ermittelt wird.

2. Verfahren nach Anspruch 1,
wobei die Teilmenge (M) mit einem elektromagnetischen Messsignal bestrahlt wird, insbesondere mit Licht.

3. Verfahren nach Anspruch 1,
wobei die Teilmenge (M) mit einem akustischen Messsignal bestrahlt wird, insbesondere mit Ultraschall.

4. Verfahren nach einem der vorhergehenden Ansprüche,
wobei der Druck (p) und/oder die Temperatur (T) in der Messzelle (12) derart angesteuert werden, dass sie oberhalb des Siedepunkts bleiben.

5. Verfahren nach einem der vorhergehenden Ansprüche,
wobei der Schwellwert (SW) als Zahlenwert vorgegeben wird.

6. Verfahren nach einem der vorhergehenden Ansprüche,
wobei bevor der Unterdruck eingestellt wird, eine Messung mit dem wellenförmigen Messsignal zur Festlegung eines Referenzwertes (RW) durchgeführt wird.

7. Verfahren nach Anspruch 6,
wobei der Referenzwert (RW) mit einem Grenzwert (GW) verglichen wird.

8. Verfahren nach Anspruch 6 oder 7,
wobei der Schwellwert (SW) auf Grundlage des Referenzwertes (RW) berechnet wird.

9. Verfahren nach einem der vorhergehenden Ansprüche,
wobei zur Bestimmung des Trübungswerts (TW) eine Intensität des wellenförmigen Messsignals nach dem Kontakt mit der Flüssigkeit (K) in der Messzelle (12) herangezogen wird.

10. Verfahren nach einem der vorhergehenden Ansprüche,
wobei ein durch die Flüssigkeit (K) transmittierter Anteil des wellenförmigen Messsignals gemessen wird.

11. Verfahren nach einem der vorhergehenden Ansprüche,
wobei ein von der Flüssigkeit (K) reflektierter Anteil des wellenförmigen Messsignals gemessen wird.

12. Verfahren nach einem der vorhergehenden Ansprüche,
wobei eine Geschwindigkeit der Flüssigkeit (K) derart geregelt wird, dass Gasblasen von einer Wand der Messzelle (12) ausgeschwemmt werden.

13. Vorrichtung (2) zur Messung eines Gasgehalts in einer Flüssigkeit (K), umfassend:
- eine durchsichtige Messzelle (12) zur Aufnahme zumindest einer Teilmenge (M) der Flüssigkeit (K),
- Mittel zum Erzeugen von Unterdruck in der Messzelle (12),
- ein Messsignalsender (6) zum Bestrahlen der Teilmenge (M) mit einem wellenförmigen Messsignal,
- mindestens einen Detektor (8, 10) zum Messen des wellenförmigen Messsignals nach dem Kontakt mit der Teilmenge (M) in der Messzelle (12),
- Sensoren (20, 22) zum Bestimmen eines Druck (p) und einer Temperatur (T) in der Messzelle (12),
- sowie eine Analyse- und Steuereinheit (18), die dafür eingerichtet ist einen Trübungswert (TW) der Flüssigkeit (K) zu bestimmen und diesen mit einem Schwellwert (SW) zu vergleichen und wenn der Trübungswert (TW) größer oder gleich dem Schwellwert (SW) ist, mit Hilfe der Druck- und Temperaturmessung in der Messzelle (12) den Gasgehalt in der Flüssigkeit (K) zu ermitteln.

14. Verwendung einer Vorrichtung (2) nach Anspruch 13 zur Messung des Gasgehaltes in einem Kühlmittel (K) einer elektrochemischen Zelle, insbesondere einer Brennstoffzelle.
